# FASCICULE DE BREVET EUROPEEN

(11) **EP 0 837 684 B1**
(45) Date de publication et mention de la délivrance du brevet: **17.04.2002**
(21) Numéro de dépôt: 96922098.7
(22) Date de dépôt: 12.06.1996
(51) Int. Cl.: A61K 31/70, A61K 31/195

(54) **COMPOSITIONS PHARMACEUTIQUES A BASE D'UN SEL DE DICLOFENAC ET DE THIOCOLCHICOSIDE**
PHARMAZEUTISCHES PRAEPARAT AUF DER BASIS EINES DICLOFENAC-SALZES UND DER VERBINDUNG THIOCOLCHICOSID
PHARMACEUTICAL COMPOSITIONS CONTAINING A DICLOFENAC SALT AND THIOCOLCHICOSIDE

(30) Priorité: 13.06.1995 FR 9506949
(43) Date de publication de la demande: 29.04.1998
(73) Titulaire: SANOFI-SYNTHELABO, 75013 Paris (FR)
(72) Inventeur: POUCHAIN, Denis, F-94300 Vincennes (FR); JACOB, Etienne, F-78150 Le Chesnay (FR); LEWIS, Gareth, F-91410 Dourdan (FR); MONTEL, Jean, F-78400 Chatou (FR)
(74) Mandataire: Thouret-Lemaitre, Elisabeth
(86) Numéro de dépôt international: FR9600885
(87) Numéro de publication internationale: WO9641635

(56) Documents cités:
- EP-A- 0 324 981
- WO-A-86/03681
- FR-M- 2 929
- GB-A- 2 218 633
- BOLL. CHIM. FARM., 1993, 132/6 (203-209), ITALY, XP002013035 PIFFERI G.: "Chemico-physical compatibility of thio-colchicoside with nonsteroidic anti-inflammatory drugs" cité dans la demande
- MINERVA ANESTESIOL, OCT 1991, 57 (10) P1084-5, ITALY, XP002013036 PALERMO S ET AL: "TENS + mesotherapy association in the therapy of cervico-brachialgia: preliminary data" cité dans la demande
- RHUMATOLOGIE (PARIS), 1983, 13/1 (45-46), FRANCE, XP002013037 ABBE R.: "Pharmacotherapy of torticollis" cité dans la demande

## Description

La présente invention a pour objet des compositions pharmaceutiques stables, sous forme solide, comprenant un sel de l'acide 2-[(2,6-dichlorophényl) amino]benzèneacétique (sel de diclofénac) et du (*S*)-N-[5,6,7,9-tétrahydro-1,3-diméthoxy-2-glucosidoxy-10-méthylthio-9-oxobenzo[*a*]heptalèn-7-yl]acétamide (thiocolchicoside), en association avec au moins un excipient pharmaceutiquement acceptable, et leur application en thérapeutique.

On connait de la demande internationale WO 86/03681, des compositions pharmaceutiques comprenant au moins un composé anti-inflammatoire non-stéroïdien (AINS) en combinaison avec un composé myorelaxant. Le diclofénac est l'un des nombreux AINS cité dans la demande internationale.
Les composés myorelaxants cités appartiennent notamment aux groupes chimiques des glycérylmonoethers, des oxazoles ,des alcanediols substitués, des benzazoles, des benzodiazépines. Tous ces composés présentent pour inconvénient d'être sédatifs.
C'est l'un des buts de l'invention décrite dans la demande internationale que de diminuer l'effet sédatif desdits myorelaxants en les associant à certains AINS. Il n'en reste pas moins que l'effet sédatif conféré par les composés myorelaxants demeurent encore.
C'est d'ailleurs pourquoi il est prévu d'associer aux composés myorelaxants et AINS, un tiers composé stimulant nerveux central, à savoir la xanthine ou un dérivé de la xanthine, telle la caféine. Ces derniers ont pour effet de compenser l'effet sédatif constaté des myorelaxants. Cependant la xanthine et ces dérivés ont pour inconvénient d'induire des effets secondaires tels les troubles du rythme et la diurèse.
Il a été proposé par S. Palermo et al., Minerva Anestesiologica, Oct. 1991, pages 1084 et 1085, une association entre du diclofénac de sodium et du thiocolchicoside sous forme d'une solution injectable.

Toutefois, il a pu être constaté par la demanderesse que la stabilité dans le temps d'une telle solution n'est pas suffisante pour envisager des préparations industrielles, prêtes à l'emploi.
Cette stabilité a été étudiée par G. Pifferi, Boll. Chim. Farmaceutico, Anno 132, N° 6, juin 1993, pages 203 à 209. Outre le problème de leur stabilité dans le temps, les solutions injectables proposées par S. Palermo présentent encore l'inconvénient d'être clouloureuse pour le patient chez qui elles sont injectées.
C'est d'ailleurs pour cette raison que ces solutions comprennent également un anésthésique local tel la lidocaïne.

On connait encore de R. Abbe "R" - 1983 - N° 61-1, pages 45 et 46, un traitement médicamenteux du torticolis discal. Ce traitement consiste à associer un salicylé ou un anti-inflammatoire à un myorelexant.
Toutefois, R. Abbe ne décrit pas l'association de ces composés en une seule et unique composition pharmaceutique stable, ni l'association spécifique d'un sel de diclofénac avec le thiocolchicoside.

La présente invention a alors pour objet une composition pharmaceutique stable comprenant un AINS et un myorelaxant obviant les inconvénients mentionnés plus haut.

Plus particulièrement, l'invention consiste en des compositions pharmaceutiques comprenant un AINS et un myorelaxant, d'administration simple et sans douleur, n'induisant aucun effet sédatif tout en permettant un effet antalgique et anti-inflammatoire renforcé.

La composition pharmaceutique selon l'invention permet encore de réduire les effets secondaires des AINS.

L'invention consiste ainsi en une composition stable, sous forme solide, comprenant un sel de diclofénac et du thiocolchicoside, en association avec au moins un excipient pharmaceutiquement acceptable.

Dans le cadre de la présente invention, on entend par le terme "forme solide", aussi bien des formes solides proprement dites, comme des comprimés, des micrograins,ou granules, des poudres et des gélules, que des formes semi-solides ou pâteuse comme des suppositoires.
Les formes solides proprement dites, sont préférées. Elles sont destinées à une administration orale.
A titre de sel de diclofénac pharmaceutiquement acceptable convenant pour la mise en oeuvre de l'invention, on peut citer les sels de potassium, de calcium, d'ammonium, d'éthylamine, de diéthylammonium et de N-(2-hydroxyéthyl)pyrrolidine du diclofénac.
Le sel de potassium, et plus encore le sel de sodium sont toutefois préférés.

Comme déjà indiqué plus haut, les compositions solides selon l'invention peuvent se présenter sous forme de comprimés, de poudre, de micrograins, de gélules comprenant de petits comprimés ou des micrograins.
Les micrograins présentent habituellement un diamètre moyen compris entre 0,6 et 1,25 mm. Les petits comprimés ont généralement un diamètre inférieur à 5 mm, plus généralement compris entre 1 et 4 mm.
On peut également associer dans une même composition pharmaceutique au moins deux d'entre des petits comprimés, des micrograins et une poudre. Ces formes galéniques sont conditionnés dans une enveloppe commune, par exemple un cachet, ou de préférence une gélule.

Quand la composition pharmaceutique selon l'invention est constituée de micrograins ou de petits comprimés, chacun de ceux-ci peut comprendre un mélange intime de sel de diclofénac et de thiocolchicoside, ou bien chaque micrograins ou chaque petit comprimé est constitué soit de thiocolchicoside soit de sel de diclofénac.

De même, lorsque la composition pharmaceutique est composée d'une association de petits comprimés et/ou de micrograins et/ou de poudres, chacun d'entre eux peut lui-même être constitué d'un mélange intime de sel de diclofénac et de thiocolchicoside ou, alternativement, chacun d'entre eux ou l'un d'entre eux seulement, peut être constitué de thiocolchicoside ou de sel de diclofénac, les autres étant constitué dudit mélange intime.
Les comprimés, petits comprimés et micrograins mentionnés plus haut peuvent ou non être pelliculés ou enrobés.
Le pelliculage ou enrobage peut avoir pour but soit de protéger la muqueuse gastro-intestinale des risques dûs à une libération trop importante ou trop localisée des principes actifs. Le pelliculage ou enrobage peut également permettre de contrôler la libération du sel de diclofénac et/ou du thiocolchicoside dans l'organisme.
Le pelliculage ou enrobage peut encore assurer simultanément les deux fonctions citées ci-dessus.
Cet enrobage peut être composé de polymères à dissolution pH-dépendante, par exemple des copolymères d'acide méthacrylique ou leur mélange, ou esters de cellulose (tels que acétophtalate, acétobutyrate), qui permettent de limiter la libération des principes actifs dans l'estomac et/ou d'assurer leur libération progressive tout au long du tractus gastro-intestinal.
Il peut également être composé (i) d'un polymère imperméable tel que l'éthylcellulose (ii) d'un polymère semi-perméable tel que l'acétate de cellulose ou un polymère méthacrylique non pH dépendant, (iii) de l'association d'un polymère imperméable avec un polymère semi-perméable ou (iv) de la combinaison de ces polymères avec des polymères à dissolution pH-dépendante.

Le pelliculage ou enrobage des comprimés et micrograins peut être réalisée classiquement par pulvérisation d'une solution de pelliculage dans un lit d'air fluidisé ou en turbine.

Les comprimés selon l'invention peuvent comprendre, outre les deux principes actifs, l'un ou plusieurs des excipients suivants :
- des diluants tels que sucres, sucre-alcools, amidon, celluloses, phosphate de calcium,
- des lubrifiants tels que l'acide stéarique, le stéarate de magnésium, les huiles hydrogénées, les acides gras, les cires, les glycérides naturels ou synthétiques,
- des éléments matriciels tels que huiles hydrogénées, glycérides, dérivés cellulosiques (méthylcellulose, carboxyméthylcellulose, éthylcellulose, hydroxyéthylcellulose, hydroxypropylméthylcellulose), dérivés vinyliques (polyvinylpyrrolidone, alcool polyvinylique), dérivés acryliques (carbomère, acrylates, méthacrylates), gommes naturelles ou synthétiques (xanthane, scléroglucane, guar),
- des agents délitants tels que carboxyméthylamidon sodique, crospovidone, carboxyméthylcelluloses réticulées,
- et des agents d'écoulement tels que silice, talc, polytétrafluoréthylène.

Les comprimés peuvent être préparés de manière connue de l'homme du métier, par mélange du ou des principes actifs avec l'un au moins des excipients tels ceux mentionnés ci-dessus, suivi ou non d'une granulation préalable du mélange obtenu, puis compression.

Les micrograins peuvent comprendre, outre les principes actifs, des excipients pharmaceutiquement acceptables tels que la cellulose, la carboxyméthylcellulose, le mannitol et la polyvinylpyrrolidone.
Les micrograins peuvent être transformés en comprimés, ou, avantageusement, conditionnés dans des gélules.

Les micrograins peuvent être préparés de façon conventionnelle pour l'homme du métier. Par exemple, on peut les préparer par enrobages successifs en turbine, d'un cristal de saccharose ou de non pareilles, par exemple en saccharose. Les couches d'enrobages peuvent être constitués de sucres, d'amidon, de celluloses ou de polyvinylpyrrolidone.
De préférence, on prépare les micrograins par mélange des excipients et des principes actifs, on humidifie le mélange obtenu, on l'extrude et on procède à une sphéronisation.

Les couches successives sont saupoudrées de poudre de sel de diclofénac et/ou de thiocolchicoside, puis séchées.

Selon un aspect particulièrement avantageux de l'invention, la composition pharmaceutique se présente sous une forme permettant la libération prolongée soit du sel de diclofénac, soit du thiocolchicoside, soit encore des deux composés. De préférence toutefois, seul le sel de diclofénac est à libération prolongée, le thiocolchicoside étant à libération rapide ou immédiate.
La forme à libération prolongée peut se présenter d'une façon classique pour l'homme du métier. Ainsi, le ou les principes actifs peuvent être sous forme de micrograins et/ou de comprimés pelliculés avec un pelliculage tel que décrit plus haut.

Plus particulièrement, la composition pharmaceutique à libération prolongée peut comprendre (i) des petits comprimés ou des micrograins de sel de diclofénac, recouverts d'une pellicule assurant la libération prolongée, notamment pH dépendante, et/ou progressive dudit sel, et (ii) des petits comprimés ou des micrograins à libération rapide ou immédiate du thiocolchicoside, non pelliculés ou recouverts d'une pellicule assurant la protection de la muqueuse gastrique vis-à-vis de ce principe actif.

Cette dernière forme a pour avantage une très bonne stabilité des principes actifs, une faible variabilité intra-individuelle ou interindividuelle tout au long de sa progression le long du tractus gastro-intestinale, ainsi qu'une plus faible agressivité vis-à-vis des muqueuses du tube digestif.

Selon un autre aspect, la composition pharmaceutique peut se présenter comme un mélange de deux groupes de micrograins ou de petits comprimés constitués chacun d'un mélange intime de sel de diclofénac et de thiocolchicoside, les micrograins ou petits comprimés d'un groupe étant pelliculés en vue d'une libération prolongée des deux principes actifs, les micrograins ou petits comprimés de l'autre groupe étant aptes à une libération rapide ou immédiate des deux principes actifs.
En faisant varier la proportion relative des micrograins ou des petits comprimés à libération prolongée et des micrograins ou des petits comprimés à libération rapide ou immédiate, on peut alors facilement contrôler la vitesse et le taux de libération de chacun des principes actifs.

Les compositions pharmaceutiques sous forme semi-solides ou pâteuses selon l'invention, peuvent être destinées à l'administration rectale ou topique. Dans ce dernier cas elles peuvent être sous forme de pommades, crèmes ou gels .

Les compositions destinées à l'administration rectale sont présentées généralement sous forme de suppositoire ou sous forme de capsules ou de gels.
Les suppositoires sont constitués habituellement de glycérides semi-synthétiques ou de polyéthylèneglycol de masse moléculaire élevée.

Les compositions pharmaceutiques selon l'invention sont présentées sous des formes contenant 25 à 150 mg de sel de sodium du diclofénac et 2 à 16 mg de thiocolchicoside par dose unitaire (1 comprimé, 1 gélule, 1 suppositoire, 1 capsule, 10 g de gel). Ces compositions pharmaceutiques peuvent être administrées à raison de 1 à 3 prises ou applications par jour.
Elles sont utilisées dans le traitement des pathologies mécaniques ou dégénératives du rachis associant une composante inflammatoire et une contracture musculaire. Elles peuvent en particulier être utilisées dans le traitement des lombalgies, de l'arthrose dorsale et cervicale, des radiculalgies et des névralgies cervico-brachiales.

Les exemples qui suivent ont pour but d'illustrer la présente invention.
Dans ces exemples, le diamètre moyen des micrograins est de 1 mm.

### Exemple 1 : comprimés à libération rapide

| | % en poids |
|---|---|
| Diclofénac, sel de sodium | 15 à 25 |
| Thiocolchicoside | 2 à 5 |
| Lactose | 20 à 48,5 |
| Cellulose microcristalline | 30 à 58,5 |
| Polyvinylpyrrolidone | 2 à 10 |
| Carboxyméthylamidon sodique | 2 à 10 |
| Stéarate de magnésium | 0,5 à 2 |

### Exemple 2 : micrograins pour gélules à libération rapide

| | % en poids |
|---|---|
| Diclofénac, sel de sodium | 15 à 25 |
| Thiocolchicoside | 2 à 5 |
| Mannitol | 10 à 39 |
| Cellulose microcristalline | 10 à 69 |
| Polyvinylpyrrolidone | 2 à 10 |
| Carboxyméthylamidon sodique | 2 à 10 |

Chacun des micrograins comprend un mélange intime de sel de sodium du diclofénac et du thiocolchicoside.

### Exemple 3 : comprimés pelliculés, avec protection gastrique

| Comprimés selon l'exemple 1 | |
|---|---|
| Pelliculage : | |
| Copolymères d'acide méthacrylique | 70 à 85 |
| Monoglycérides diacétylés | 5 à 10 |
| Talc | 10 à 20 |

La quantité de pelliculage déposé sur les comprimés correspond à 1 à 8 % en poids sec par rapport au poids total des comprimés.

### Exemple 4 : comprimés à libération prolongée, avec protection gastrique

| Comprimés selon l'exemple 1 | |
|---|---|
| Pelliculage : | |
| Copolymères d'acide méthacrylique | 50 à 85 |
| Ethylcellulose | 20 à 40 |
| Monoglycérides diacétylés | 5 à 10 |

La quantité de pelliculage déposé sur les comprimés correspond à 1 à 8 % en poids sec par rapport au poids total des comprimés.

### Exemple 5 : gélules à libération prolongée du diclofénac et à libération rapide du thiocolchicoside

| Micrograins pelliculés : | |
|---|---|
| | % en poids |
| Micrograins : | |
| Diclofénac, sel de sodium | 20 |
| Lactose | 22 |
| Cellulose microcristalline | 53 |
| Polyvinylpyrrolidone | 5 |

| Pelliculage : | |
|---|---|
| Copolymères d'acide méthacrylique | 62 |
| Ethylcellulose | 30 |
| Monoglycérides diacétylés | 8 |

Le mélange des constituants des micrograins est extrudé après humidification, puis sphéronisé. Les micrograins obtenus sont séchés puis pelliculés par la composition de pelliculage définie ci-dessus, à raison de 2 à 10 % de leur masse.

| Micrograins : | |
|---|---|
| Thiocolchicoside | 5 |
| Lactose | 38 |
| Cellulose microcristalline | 46 |
| Polyvinylpyrrolidone | 5 |
| Carboxyméthylamidon sodique | 6 |

Les micrograins pelliculés contenant le diclofénac sont mélangés avec les micrograins de thiocolchicoside de façon à obtenir les doses unitaires souhaitées de chacun des principes actifs, puis le mélange est réparti en gélules.

### Exemple 6 : micrograins à libération prolongée, avec protection gastrique

| | |
|---|---|
| Micrograins selon l'exemple 2 | 5 à 20 |
| Micrograins selon l'exemple 2 et Pellicules selon l'exemple 4 | 75 à 94,5 |
| Talc | 0,5 à 5 |
| Le talc permet d'éviter que les micrograins n'adhèrent entre eux. | |

### Exemple 7 : comprimés à libération prolongée

| | % en poids |
|---|---|
| Diclofénac, sel de sodium | 20 |
| Thiocolchicoside | 1,6 |
| Lactose | 30 |
| Cellulose microcristalline | 26,9 |
| Huile de ricin hydrogénée | 15 |
| Hydroxypropylméthylcellulose | 5 |
| Silice colloïdale | 0,5 |
| Stéarate de magnésium | 1 |

### Exemple 8 : gel topique

| | |
|---|---|
| Diclofénac, sel de diéthylammonium | 1000 mg |
| Thiocolchicoside | 50 mg |
| Propylèneglycol | 25 g |
| Carbomère 940 | 5 g |
| Hydroxyde de sodium | qsp pH 7,8 à 8 |
| Eau purifiée | qsp 100 g |

### Exemple 9 : suppositoire

| | |
|---|---|
| Diclofénac, sel de sodium | 100 mg |
| Thiocolchicoside | 5 mg |
| Triglycérides semi-synthétiques d'acides gras insaturés | 1800 mg |

## Revendications

1. Composition pharmaceutique **caractérisée en ce qu'**elle comprend un sel de diclofénac et du thiocolchicoside, en association avec au moins un excipient pharmaceutiquement acceptable, ladite composition pharmaceutique se présentant sous une forme solide, stable dans le temps.

2. Composition pharmaceutique selon la revendication 1, **caractérisée en ce que** le sel de diclofénac est le sel de sodium du diclofénac.

3. Composition pharmaceutique selon l'une des revendications 1 et 2, **caractérisée en ce qu'**elle est destinée à une administration orale.

4. Composition pharmaceutique selon l'une des revendications 1 à 3, **caractérisée en ce qu'**elle se présente sous une forme à libération prolongée du sel de diclofénac, du thiocolchicoside ou du sel de diclofénac et du thiocolchicoside.

5. Composition pharmaceutique selon l'une des revendicationS 1 à 4, **caractérisée en ce qu'**eale comprend un mélange(i) de micrograins ou petits comprimés de thiocolchicoside et (ii) de micrograins ou de petits comprimés de sel de diclofénac.

6. Composition pharmaceutique selon la revendication 5, **caractérisée en ce que** les micrograins ou petits comprimés de sel de diclofénac se présentent sous une forme à libération prolongée.

7. Composition pharmaceutique selon l'une des revendications 5 et 6, **caractérisée en ce que** les micrograins ou petits comprimés de thiocolchicoside se présentent sous une forme à libération rapide ou immédiate.

8. Composition pharmaceutique selon l'une des revendications 1 à 4, **caractérisée en ce qu'**elle se présente sous forme de comprimé, de gel, de pommade ou de suppositoire.

9. Composition pharmaceutique selon l'une quelconque des revendications 1 à 8, **caractérisée en ce qu'**elle est présentée sous une forme contenant 25 à 150 mg de sel de diclofénac et 2 à 16 mg de thiocolchicoside par dose unitaire.

## Claims

1. Pharmaceutical composition, **characterized in that** it comprises a diclofenac salt and thiocolchicoside, in combination with at least one pharmaceutically acceptable excipient, the said pharmaceutical composition being provided in a solid form which is stable over time.

2. Pharmaceutical Composition according to Claim 1, **characterized in that** the diclofenac salt is the sodium salt of diclofenac.

3. Pharmaceutical composition according to either of Claims 1 and 2, **characterized in that** it is intended for oral administration.

4. Pharmaceutical composition according to one of Claims 1 to 3, **characterized in that** it is provided in a form for prolonged release of the diclofenac salt, the thiocolchicoside or the diclofenac salt and the thiocolchicoside.

5. Pharmaceutical composition according to one of Claims 1 to 4, **characterized in that** it comprises a mixture (i) of micrograins or small tablets of thiocolchicoside and (ii) of micrograins or small tablets of diclofenac salt.

6. Pharmaceutical composition according to Claim 5, **characterized in that** the micrograins or small tablets of diclofenac salt are provided in a prolonged release form.

7. Pharmaceutical composition according to either of Claims 5 and 6, **characterized in that** the micrograins or small tablets of thiocolchicoside are provided in a rapid or immediate release form.

8. Pharmaceutical composition according to one of Claime 1 to 4, **characterized in that** it is provided in the form of a tablet, gel, ointment or suppository.

9. Pharmaceutical composition according to any one of Claims 1 to 8, **characterized in that** it is provided in a form containing 25 to 150 mg of diclofenac salt and 2 to 16 mg of thiocolchicoside per unit dose.

## Patentansprüche

1. Pharmazeutische Zubereitung, **dadurch gekennzeichnet, daß** sie ein Salz von Diclofenac und Thiocolchicosid in Kombination mit mindestens einem pharmazeutisch annehmbaren Trägermaterial umfaßt, wobei die pharmazeutische Zubereitung in fester, zeitlich stabiler Form vorliegt.

2. Pharmazeutische Zubereitung nach Anspruch 1, **dadurch gekennzeichnet, daß** das Salz von Diclofenac das Natriumsalz von Diclofenac ist.

3. Pharmazeutische Zubereitung nach einem der Ansprüche 1 und 2, **dadurch gekennzeichnet, daß** sie auf oralem Wege verabreichbar ist.

4. Pharmazeutische Zubereitung nach einem der Ansprüche 1 bis 3, **dadurch gekennzeichnet, daß** sie in einer Form mit verzögerter Freisetzung des Salzes von Diclofenac, von Thiocolchicosid oder des Salzes von Diclofenac und Thiocolchicolsid vorliegt.

5. Pharmazeutische Zubereitung nach einem der Ansprüche 1 bis 4, **dadurch gekennzeichnet, daß** sie eine Mischung (i) von Mikrokörnchen oder kleinen Tabletten von Thiocolchicosid und (ii) Mikrokörnchen oder kleinen Tabletten des Salzes von Diclofenac umfaßt.

6. Pharmazeutische Zubereitung nach Anspruch 5, **dadurch gekennzeichnet, daß** die Mikrokörnchen und die kleinen Tabletten des Salzes von Diclofenac in einer Form für die verzögerte Wirkstofffreisetzung vorliegen.

7. Pharmazeutische Zubereitung nach einem der Ansprüche 5 und 6, **dadurch gekennzeichnet, daß** die Mikrokörnchen oder die kleinen Tabletten von Thiocolchicosid in einer Form für die schnelle oder sofortige Wirkstofffreisetzung vorliegen.

8. Pharmazeutische Zubereitung nach einem der Ansprüche 1 bis 4, **dadurch gekennzeichnet, daß** sie in Form einer Tablette, eines Gels, einer Salbe oder eines Suppositoriums vorliegt.

9. Pharmazeutische Zubereitung nach einem der Ansprüche 1 bis 8, **dadurch gekennzeichnet, daß** sie in einer Form vorliegt, die 25 bis 150 mg des Salzes von Diclofenac und 2 bis 16 mg Thiocolchicosid pro Einheitsdosis enthält.
